# EUROPÄISCHE PATENTSCHRIFT

(11) **EP 1 496 852 B1**
(45) Veröffentlichungstag und Bekanntmachung des Hinweises auf die Patenterteilung: **10.10.2007**
(21) Anmeldenummer: 03717289.7
(22) Anmeldetag: 10.04.2003
(51) Int. Cl.: A61Q 17/04, B05B 11/00, B05B 7/00

(54) **SONNENSCHUTZEMULSION MIT SCHAUMSPENDER**
SUN PROTECTING EMULSION PROVIDED WITH A FOAM DISPENSER
EMULSION DE PROTECTION SOLAIRE ET DISTRIBUTEUR DE MOUSSE

(30) Priorität: 19.04.2002 DE 10217474
(43) Veröffentlichungstag der Anmeldung: 19.01.2005
(73) Patentinhaber: Cognis IP Management GmbH, 40589 Düsseldorf (DE)
(72) Erfinder: KAWA, Rolf, 40789 Monheim (DE); FINKEL, Peter, 51519 Odenthal (DE); MERHEIM, Hannelore, 51149 Köln (DE); HENIG, Sylvia, 51371 Leverkusen (DE)
(86) Internationale Anmeldenummer: PCT/EP2003/003711
(87) Internationale Veröffentlichungsnummer: WO 2003/088941

(56) Entgegenhaltungen:
- EP-A- 0 860 164
- EP-A- 0 878 469
- EP-A- 1 316 300
- WO-A-00/36063
- WO-A-00/78629
- WO-A-99/11235
- WO-A-99/67016
- DE-A- 19 955 375
- US-B1- 6 368 577
- DATABASE PROMT [Online] STN INTERNATIONAL, KARLSRUHE; SEMENCZUK, S.; CHAZALY, C. ET AL.: "Synergistic Sun System" Database accession no. 1999:271710 XP002245496 & SOAP & COSMETICS, Bd. 75, Nr. 4, April 1999 (1999-04), Seite 48(1)
- AIRSPRAY INT. B.V.: HOMEPAGE, [Online] XP002245495 Gefunden im Internet: <URL:http://www.airspray.nl/TurboBrown.htm l> [gefunden am 2003-06-25]

## Beschreibung

### Gebiet der Erfindung

Gegenstand der vorliegenden Anmeldung sind verschäumbare kosmetische und/oder pharmazeutische Sonnenschutzemulsionen in einem Schaumspender.

### Stand der Technik

Obwohl Emulsionen seit langer Zeit bekannt sind, bestehen auf dem Kosmetikmarkt intensive Bemühungen insbesondere die sensorischen Eigenschaften dieser dispersen Systeme stets zu verbessern, da gerade ungünstige rheologische Eigenschaften, wie z.B. Rheopexie, ein gleichmäßiges Verteilen erschweren. Gegenwärtig werden vermehrt tensidhaltige Emulsionen, die durch einen speziellen Pumpmechanismus mit Luft verschäumt werden, im Markt angeboten. Aufgrund der geringeren Dichte des Schaums lassen sich derartige Systeme leichter und gleichmäßiger auf der Haut verteilen. In der praktischen Anwendung zeigt sich jedoch, dass der Schaum solcher Emulsionen, wie beispielsweise in der DE 199 55 375 A1 beschrieben, von zu geringer Festigkeit und Intensität ist, sehr schnell bricht und einen wässrigen, sensorisch nicht völlig zufriedenstellenden Eindruck auf der Haut hinterläßt. Eine gleichmäßige und homogene Verteilung dieser Schäume auf der Haut ist nicht immer gewährleistet. Für spezielle Applikationsbereiche, wie beispielsweise Sonnenschutzemulsion, ist die homogene Verteilung des Mittels jedoch besonders wichtig, um eine gleichmäßige und homogene Verteilung der eingesetzten UV-Lichtschutzfilter auf der Haut und damit eine reproduzierbare Sonnenschutzleistung zu erzielen.

Aufgabe war es daher, Sonnenschutzemulsionen zur Verfügung zu stellen, die sich leicht mit einem Gas, insbesondere mit Luft, verschäumen lassen und einen stabilen Schaum bilden, der leicht und gleichmäßig verteilbar ist und ein sensorisch leichtes Hautgefühl vermittelt. Ein weiterer Aspekt der Aufgabe war es, besonders hautverträgliche, verschäumbare Sonnenschutzemulsionen zur Verfügung zu stellen.

Das Patent EP-878469 wird als nächstliegender Stand der Technik gegenüber dem Gegenstand der Anspruch 1 angesehen. Es offenbart (siehe Beispiele 8) einen Schaum in einen Aerosolbehälter, **1%** Poly(12-hydroxystearinsäure)- polyglycerinester neben 83,3 Gew.% Wasser, einem UV-Lichtschutzfilter, 0,5% nonionisch Tenside(Oleth 20) und einem Olkörper enthalt, und abgefullt und mit Treibgas versetzt wird.
Und in WO-0036063 wird Poly(12-hydroxystearinsaure)-polyglycerinester wie ein Emulgator Bestandteil neben ein Schaumregulator offenbart.

### Beschreibung der Erfindung

Überraschenderweise wurde gefunden, dass Sonnenschutzemulsionen auf Basis von Polyolpoly-12-Hydroxystearaten als Emulgator besonders leicht verschäumbar sind und sehr stabile und hautverträgliche Schäume bilden, die eine sehr homogene Verteilung der UV-Lichtschutzfilter ermöglichen.

Gegenstand der vorliegenden Erfindung ist daher ein System aus einem manuell betätigbarem Schaumspender und einer Sonnenschutzemulsion, gekennzeichnet durch einen Gehalt an:
(a) Polyolpoly-12-Hydroxystearaten
(b) Ölkörpern
(c) weiteren Tensiden
(d) UV-Lichtschutzfiltern und
(e) Wasser

Gegenstand der Erfindung sind auch die Sonnenschutzemulsionen selbst. Die verschäumbare Sonnenschutzemulsion enthält üblicherweise 30 - 80 Gew.-%, vorzugsweise 50 - 80 Gew.-% und insbesondere 60 - 80 Gew.-% Wasser.

Die im Schaumspender bevorratete Sonnenschutzemulsion enthält vorzugsweise (a) 2 - 10 Gew.-% Polyolpoly-12-Hydroxystearate, (b) 1- 20 Gew.-% Ölkörper, (c) 0,5-10 Gew.-% Tenside und (d) 0,5 - 20 Gew.-% UV-Lichtschutzfilter und (d) 30 - 80 Gew.-% Wasser. In einer bevorzugten Ausführungsform handelt es sich dabei um W/O-Sonnenschutzemulsionen. Ein weiterer Gegenstand der vorliegenden Erfindung ist die Verwendung von Polyolpoly-12-Hydroxystearaten, insbesondere von Poly(12-hydroxystearinsäure)polyglycerinestern zur Verbesserung der Verschäumbarkeit von Emulsionen in Schaumspendern.

### Polyolpoly-12-Hydroxystearate (Komponente a)

Bei den Polyolpoly-12-hydroxystearaten, welche die Komponente (a) bilden, handelt es sich um bekannte Stoffe, die beispielsweise unter den Marken "Dehymuls^{®} PGPH" oder "Eumulgin^{®} VL 75" (Abmischung mit Coco Glucosides im Gewichtsverhältnis 1:1) oder Dehymuls^{®} SBL von der Cognis Deutschland GmbH vertrieben werden. In diesem Zusammenhang sei insbesondere auf das Europäische Patent EP 0 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
➢ Glycerin und Polyglycerin;
➢ Alkylenglycole, wie beispielsweise Ethylenglycol, Diethylenglycol, Propylenglycol;
➢ Methylolverbindungen, wie insbesondere Trimethylolethan, Trimethylolpropan, Trimethylolbutan, Pentaerythrit und Dipentaerythrit;
➢ Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit;
➢ Zucker mit 5 bis 12 Kohlenstoffatomen, wie beispielsweise Glucose oder Saccharose;
➢ Aminozucker, wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft haben sich Umsetzungsprodukte von Poly-12-hydroxystearinsäure mit Polyglycerinen folgender Homologenverteilung erwiesen (bevorzugte Mengen sind in Klammern angegeben):

| | |
|---|---|
| Glycerine | : 5 bis 35 (15 bis 30) Gew.-% |
| Diglycerine | : 15 bis 40 (20 bis 32) Gew.-% |
| Triglycerine | : 10 bis 35 (15 bis 25) Gew.-% |
| Tetraglycerine | : 5 bis 20 (8 bis 15) Gew.-% |
| Pentaglycerine | : 2 bis 10 (3 bis 8) Gew.-% |
| Oligoglycerine | : ad 100 Gew.-% |

In einer bevorzugten Ausführungsform der Erfindung ist als Polyolpoly-12-Hydroxystearat wenigstens ein Poly(12-hydroxystearinsäure)polyglycerinester enthalten, der beispielsweise von der Cognis Deutschland GmbH & Co. KG unter der Bezeichnung Dehymuls^{®} PGPH vermarktet wird und einen W/O-Emulgator darstellt. Dieser kann in den erfindungsgemäßen Sonnenschutzemulsionen üblicherweise in einer Menge von 0,5-10 Gew.-% vorhanden sein; erfindungsgemäß bevorzugt ist eine Menge von 2 -10 Gew.-% und insbesondere 3 - 8 Gew.%.

### Ölkörper (Komponente b)

Unter Ölkörpern, welche die Komponente (b) bilden, sind erfindungsgemäß für kosmetische Applikationen geeignete, bei 20 °C flüssige, mit Wasser bei-25 °C nicht mischbare Stoffe oder Stoffgemische zu verstehen. Je nach Applikationsform (z. B. W/O oder O/W-Emulsion) und der eingesetzten UV-Lichtschutzfilter (flüssig oder kristallin) kann die Menge der Ölkörper an der Gesamtzusammensetzung zwischen 1 und 20 Gew.-% variieren, wobei bei flüssigen UV-Lichtschutzfiltern auch mit einer Untergrenze von 1 Gew.-% Ölkörpern gearbeitet werden kann. Die Ölkörper, bei denen es sich sowohl um Reinsubstanzen als auch um Substanzgemische handeln kann, werden üblicherweise in einer Menge von 1 - 20 Gew.-%, vorzugsweise 1 - 15 Gew.-% und insbesondere 5 - 10 Gew.-% eingesetzt. Ein Gemisch aus Ölkörpern ist erfindungsgemäß bevorzugt.

Als Ölkörper kommen beispielsweise Guerbetalkohole auf Basis von Fettalkoholen mit 6 bis 18, vorzugsweise 8 bis 10 Kohlenstoffatomen (z. B. Eutanol^{®} G), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₃-C₃₈-Alkylhydroxycarbonsäuren mit linearen oder verzweigten C₆-C₂₂-Fettalkoholen - insbesondere Diethylhexylmalat-, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C-Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen (Hydagen^{®} HSP, Sovermol^{®} 750, Sovermol^{®} 1102), Siliconöle (Cyclomethicone, Siliciummethicontypen u.a.) und/oder aliphatische bzw. naphthenische Kohlenwasserstoffe, wie z.B. Mineralöl, Vaseline, Petrolatum, Squalan, Squalen, Isohexadecane oder Dialkylcyclohexane in Betracht.

Sensorisch besonders elegante Schäume werden erhalten, wenn als Ölkörper Dialkylether und/oder Dialkylcarbonate eingesetzt oder mitverwendet werden. Erfindungsgemäß sind diese daher als Ölkörper bevorzugt. Eine besonders bevorzugte Ölkörper-Kombination enthält Triglyceride und Dialkylcarbonate, vorzugsweise eine Mischung aus Dicaprylyl Carbonate (Cetiol^{®} CC) und Cocoglyceride (Myritol^{®} 331) und/oder Caprylic/Capric Triglyceride (Myritol^{®} 318). Darüber hinaus kann es bevorzugt sein, Siliconverbindungen - wie beispielsweise Cyclomethicone und Dimethicone - zu verwenden, um das unerwünschte, sogenannte "Weißeln" (Mikroschaumbildung) auf der Haut zu verhindern.

Sensorisch besonders elegante Schaume werden auch mit Ölen wie Cetiol^{®} OE (Dicaprylyl Ether), Finsolv^{®} TN (C12/15 Alkyl Benzoate), Tegosoft® OP (Ethylhexyl Palmitate) und Kombinationen dieser Öl erhalten.

### Tenside (Komponente c)

Die erfindungsgemäße Zusammensetzung enthält zur Schaumbildung und -stabilisierung weitere Tenside, die nicht unter die Komponente (a) fallen. Hierunter werden die dem Fachmann bekannten oberflächenaktiven Substanzen verstanden. Die Tenside sind in den erfindungsgemäßen Zusammensetzungen in Mengen von 0,5 -10 Gew.%, vorzugsweise 1- 8 Gew.-% und insbesondere 2 - 6 Gew.%, bezogen auf die Aktivsubstanz, enthalten. Erfindungsgemäß kann ein einziges Tensid eingesetzt werden oder ein Gemisch aus Tensiden, wobei letztere bevorzugt sind.

Prinzipiell können die Zusammensetzungen anionische, amphotere, zwitterionische, nichtionische und ferner ggf. auch kationische Tenside enthalten. In einer bevorzugten Ausführungsform ist wenigstens ein Tensid ausgewählt aus der Gruppe der Aniontenside und/oder der zwitterionischen Tenside enthalten. Bevorzugt ist eine Kombination dieser beiden Tensid-Klassen (vide infra).

Aniontenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze. Erfindungsgemäß besonders gut geeignete Aniontenside sind beispielsweise Plantapon^{®} CMGS (Sodium Hydrogenated Glyceryl Cocoate Sulfate), Plantapon^{®} ACG 35 (Sodium Cocoyl Glutamate), Hostapon^{®} CLG (Sodium Lauroyl Glutamate), Rewopol^{®} SBCS 50K (Disodium PEG-5 Laurylcitrate Sulfosuccinate, Sodium Laureth Sulfate) sowie Mischungen dieser Tenside. Für die erfindungsgemäßen Zubereitungen sind unter den anionischen Tensiden Alkalisalze von Sulfosuccinaten bevorzugt geeignet, da sie besonders hautfreundlich sind und sehr stabile und intensive Schäume liefern.

Erfindungsgemäß bevorzugt geeignet sind auch zwitterionische Tenside. Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat. Erfindunsggemäß besonders bevorzugt ist Tego^{®} Betain 810 (INCI: Capryl/Capramidopropyl Betaine) sowie eine Tensidmischung aus Rewopol^{®} SBCS 50K (INCI: Disodium PEG-5 Laurylcitrate Sulfosuccinate, Sodium Laureth Sulfate) und Tego^{®} Betain 810 (Capryl/Capramidopropyl Betaine), insbesondere im Gewichtsverhältnis 1 : 4 bis 4 : 1, ganz besonders bevorzugt im Gewichtsverhältnis von 1:4 bis 1:1.

Eine besonders bevorzugte Ausführungsform der Sonnenschutzemulsion enthält (a) 2 - 10 Gew.-% Poly(12-hydroxystearinsäure)polyglycerinester, (b) 1 - 20 Gew.-% Ölkörper enthaltend Dialkylcarbonate, (c) 0,5-10 Gew.-% eines Gemisches aus Cocamidopropylbetain und Sulfosuccinaten und (d) 0,5-20 Gew.-% UV-Lichtschutzfilter und (c) 30 - 80 Gew.-% Wasser.

Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Bevorzugte ampholytische Tenside sind das N-Kokosalkylaminopropionat, das Kokosacylaminoethylaminopropionat und das C₁₂-₁₈-Acylsarcosin.

Als kationische Tenside sind insbesondere quartäre Ammoniumverbindungen verwendbar. Tenside aus dieser Stoffklasse haben eine besonders hohe Affinität zur Haut und können den Grad der sensorischen Glätte verbessern. Hierzu zählen unter anderem Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretriethanolaminestersalze verstanden. Sie verleihen den Zusammensetzungen einen besonderen Weichgriff. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

### Nicht-ionische Emulgatoren

Die erfindungsgemäßen Zubereitungen können zusätzlich auch weitere nichtionische Emulgatoren enthalten, die nicht unter die als Komponente (a) genannten Polyolpoly-12-hydroxystearate fallen. Nichtionische Emulgatoren zeichnen sich durch ihre Hautfreundlichkeit und Milde sowie ihre ökotoxologisch guten Eigenschaften aus. Durch Verwendung einer Kombination nicht-ionischer W/O und O/W-Emulgatoren kann weiterhin Stabilität und Sensorik der erfindungsgemäßen Zusammensetzungen verbessert werden.

Zur Gruppe der nicht-ionischen Emulgatoren gehören:
➢ Anlagerungsprodukte von 2 bis 50 Mol Ethylenoxid und/oder 0 bis 20 Mol Propylenoxid an lineare Fettalkohole mit 8 bis 40 C Atomen, an Fettsäuren mit 12 bis 40 C-Atomen und an Alkylphenole mit 8 bis 15 C-Atomen in der Alkylgruppe.
➢ C_{12/18}-Fettsäuremono- und -diester von Anlagerungsprodukten von 1 bis 50 Mol Ethylenoxid an Glycerin.
➢ Glycerinmono- und -diester und Sorbitanmono- und -diester von gesättigten und ungesättigten Fettsäuren mit 6 bis 22 Kohlenstoffatomen und deren Ethylenoxidanlagerungsprodukte.
➢ Alkylmono- und -oligoglycoside mit 8 bis 22 Kohlenstoffatomen im Alkylrest und deren ethoxylierte Analoga.
➢ Anlagerungsprodukte von 7 bis 60 Mol Ethylenoxid an Ricinusöl und/oder gehärtetes Ricinusöl.
➢ Andere als die unter Komponente (a) genannten Polyol- und Polyglycerinester, wie z. B. Polyglycerindiisostearat oder Polyglycerindimerat. Ebenfalls geeignet sind Gemische von Verbindungen aus diesen Substanzklassen.
➢ Anlagerungsprodukte von 2 bis 15 Mol Ethylenoxid an Ricinusöl und/oder gehärtetes Ricinusöl.
➢ Partialester auf Basis linearer, verzweigter, ungesättigter bzw. gesättigter C₆-C₂₂-Fettsäuren, Ricinolsäure sowie 12-Hydroxystearinsäure mit Pentaerythrit, Dipentaerythrit, Zuckeralkoholen (z. B. Sorbit), Alkylglucosiden (z. B. Methylglucosid, Butylglucosid, Laurylglucosid) sowie Polyglucosiden (z. B. Cellulose), oder Mischester wie z. B. Glycerylstearatcitrat und Glycerylstearatlactat.
➢ Wollwachsalkohole.
➢ Polysiloxan-Polyalkyl-Polyether-Copolymere bzw. entsprechende Derivate.
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. Für die erfindungsgemäßen Zubereitungen sind die Umsetzungsprodukte mit 1-100 Mol Ethylenoxid besonders gut geeignet.

Erfindungsgemäß bevorzugt sind Abmischungen von Polyolpoly-12-hydroxystearaten mit anderen Emulgatoren, die beispielsweise unter der Bezeichnung Eumulgin^{®} VL 75 (O/W-Emulgator) und Dehymuls^{®} SBL (W/O-Emulgator) von der Cognis Deutschland GmbH & Co. KG vertrieben werden.

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Für ethoxylierte Produkte läßt sich der HLB-Wert auch nach folgender Formel berechnen: HLB = (100 - L): 5, wobei L der Gewichtsanteil der lipophilen Gruppen, d. h. der Fettalkyl- oder Fettacylgruppen in Gewichtsprozent, in den Ethylenoxidaddukten ist.

Vorteilhaft aus der Gruppe der W/O-Emulgatoren sind Partialester von Polyolen, insbesondere von C₃-C₆-Polyolen, wie beispielsweise Glycerylmonoestern, Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Zur Gruppe der nicht-ionischen O/W-Emulgatoren (HLB-Wert: 8-18) und/oder Solubilisatoren zählen u.a. Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren.

Nicht-ionische Emulgatoren aus der Gruppe der Alkyloligoglycoside sind besonders hautfreundlich und können daher im Sinne der Erfindung bevorzugt als O/W-Emulgatoren geeignet sein, da sie unabhängig von der Polarität des eingesetzten Ölkörpers ein besonders hohes Stabilisierungspotential, insbesondere in Kombination mit den erfindungsgemäßen Emulgatoren aufweisen. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen, vorzugsweise 12 bis 22, und besonders bevorzugt 12 bis 18 C-Atomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß vorteilhaft einsetzbar ist ein Gemisch, das unter der Bezeichnung Emulgade^{®} PL 68/50 von der Cognis Deutschland GmbH & Co. KG vertrieben und ein 1:1-Gemisch aus Alkylpolyglucosiden und Fettalkoholen darstellt.

### UV-Lichtschutzfilter (Komponente d)

Die Sonnenschutzemulsion enthält als Komponente (d) einen UV-Lichtschutzfilter oder eine Kombination von UV-Lichtschutzfiltern. Kombinationen verschiedener UV-Lichtschutzfilter sind erfindungsgemäß bevorzugt, da sie einen "Breitbandschutz" vor UV-Strahlen verschiedener Wellenlängen gewährleisten.

Erfindungsgemäß sind als UV-Lichtschutzfaktoren bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2-(1,4-Phenylene)bis-1H-benzimidazole - 4,6 disulfonsäure und ihre Salze, bevorzugt das Natriumsalz
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Günstige Kombinationen bestehen aus den Derivaten des Benzoylmethans, z. B. 4-tert.-Butyl-4'-methoxydibenzoylmethan (Parsol® 1789) und 2-Cyano-3,3-phenylzimtsäure-2-ethyl-hexylester (Octocrylene) sowie Benzylidene Malonate Polysiloxane (Parsol® SLX) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Parsol^{®} SLX ist ein bevorzugt geeigneter, öllöslicher UV-Filter für die erfindungsgemäßen Schaumemulsionen, mit dem sich ein besonders gutes Hautgefühlt des Schaums erreichen läßt. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert. Im Sinne der vorliegenden Erfindung sind Ethylhexyl Triazone - Uvinul® T 150 (2,4,6-Tris[p-(2-ethylhexyloxycarbonyl)anilino]-1,3,5-triazin, Diethylhexyl Butamido Triazone - Uvasorb® HEB (4,4'-[(6-[4-((1,1-Dimethylethyl)-amino-carbonyl)phenylamino]-1,3,5,-triazin-2,4-yl)diimino]bis-(benzoesäure-2-ethylhexylester), Octocrylene - Neo Heliopan® 303 (2-Cyan-3,3-diphenyl-acrylsäure-(2-ethylhexylester), Butyl Methoxydibenzoylmethane - Parsol® 1789 (1-(4-tert.-Butylphenyl)-3-(4-methoxyphenyl)propan-1,3-dion), Ethylhexyl Methoxycinnamat - Neo Heliopan® AV (4-Methoxy-zimtsäure-2-ethyl-hexylester) bzw. Isoamyl p-Methoxycinnamate - Neo Heliopan® E 1000 (4-Methoxy-zimtsäure-isoamylester), Homosalate - Neo Heliopan® HMS (3,3,5-Trimethyl-cyclohexyl-salicylat), Ethylhexyl Salicylate - Neo Heliopan® OS (Salicylsäure-2-ethylhexylester), 4-Methylbenzylidene Camphor - Neo Heliopan® MBC (2-(4'Methylbenzyliden)-DL-campher, Phenylbenzimidazole Sulfonic Acid - Neo Heliopan® Hydro (2-Phenylbenzimidazol-5-sulfonsäure und ihre Salze), Disodium Phenyl Dibenzimidazole Tetrasulfonate - Neo Heliopan® AP (2,2-(1,4-Phenylene)bis-(1H-benzimidazole-4,6 disulfonic acid und ihre Salze) bevorzugt.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und ferner Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) sowie Parf. Kosm. 3, 11 (1999) zu entnehmen.

Neben den beiden vorgenannten Gruppen primärer UV-Lichtschutzfilter können auch sekundäre Lichtschutzfilter vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin -und deren Glycosyl-, N-Acetyl-; Methyl-; Ethyl-, Propyl-, Amyl-,Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. y-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Erfindungsgemäß bevorzugt ist der Einsatz einer Kombination primärer UV-Lichtschutzfilter oder eine Kombination primärer und sekundärer UV-Lichtschutzfilter.

### Feuchthaltemittel/Hautbefeuchtungsmittel

In einer weiteren bevorzugten Ausführungsform enthält die Sonnenschutzemulsion auch ein Feuchthaltemittel. Dieses dient zur weiteren Optimierung der sensorischen Eigenschaften der Zusammensetzung sowie zur Feuchtigkeitsregulierung der Haut. Auch die Kältestabilität der erfindungsgemäßen Zubereitungen, insbesondere im Falle von Emulsionen, wird dadurch erhöht werden. Die Feuchthaltemittel sind üblicherweise in einer Menge von 0,1-15 Gew.-%, vorzugsweise 1-10 Gew.-%, und insbesondere 3-5 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Insektenrepellent-Wirkstoffe

Eine weitere Ausführungsform der erfindungsgemäßem Sonnenschutzemulsion kann zusätzlich Insektenrepellent-Wirkstoffe oder eine Kombination dieser Wirkstoffe enthalten.

Als Insekten-Repellentien kommen beispielsweise N,N-Diethyl-m-toluamid, 1,2-Pentandiol oder 3-(N-n-Butyl-N-acetyl-amino)-propionic acid-ethyl ester), welches unter der Bezeichnung Insect Repellent 3535 von der Merck KGaA vertrieben wird, sowie Butylacetylaminopropionate in Frage. Sie werden in den erfindungsgemäßen Zusammensetzungen üblicherweise in einer Menge von 0,1 - 10 Gew.-%, vorzugsweise 1 - 8 Gew.-%, und besonders bevorzugt in einer Menge von 2 - 6 Gew.-% bezogen auf die Gesamtzusammensetzung eingesetzt.

### Viskositätsregulatoren

Die gewünschte Viskosität der erfindungsgemäßen Zusammensetzungen kann durch Zugabe von Viskositätsregulatoren erreicht werden. Als Viskositätsregulatoren kommen u.a. Konsistenzgeber, wie z. B. Fettalkohole oder Hydroxyfettalkohole mit 12 bis 22 und vorzugsweise 16 bis 18 Kohlenstoffatomen sowie Partialglyceride, Fettsäuren mit 12 bis 22 Kohlenstoffatomen oder 12-Hydroxyfettsäuren in Betracht. Geeignet ist auch eine Kombination dieser Stoffe mit Alkyloligoglucosiden und/oder Fettsäure-N-methylglucamiden gleicher Kettenlänge, da derartige Kombinationen besonders stabile und homogene Emulsionen liefern. Zu den Viskositätsregulatoren zählen auch Verdickungsmittel wie beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), unvernetzte und mit Polyolen vernetzte Polyacrylsäuren, Polyacrylamide, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Auch Tenside, wie beispielsweise ethoxylierte Fettsäureglyceride, Ester von Fettsäuren mit Polyolen, wie beispielsweise Pentaerythrit oder Trimethylolpropan, Fettalkoholethoxylate mit eingeengter Homologenverteilung, Alkyloligoglucoside sowie Elektrolyte, wie z. B. Kochsalz und Ammoniumchlorid können zur Viskositätsregulierung eingesetzt werden.

Als Viskositätsregulatoren eignen sich auch anionische, zwitterionische, amphotere und nichtionische Copolymere wie beispielsweise Vinylacetat/Crotonsäure-Copolymere, VinylpyrrolidonNinylacrylat-Copolymere, Vinylacetat/Butylmaleat/Isobornylacrylat-Copolymere, Methylvinylether/Maleinsäureanhydrid-Copolymere und deren Ester, Acrylamidopropyltrimethylammoniumchlorid/Acrylat-Copolymere, Octylacrylamid/Methylmethacrylat/tert.-Butylaminoethylmethacrylat/2-Hydroxypropylmethacrylat-Copolymere, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone.

### Weitere Hilfs- und Zusatzstoffe (fakultativ)

Die erfindungsgemäßen Zusammensetzungen können je nach Art und Zweck der Applikation weitere Hilfs- und Zusatzstoffe enthalten, wie beispielsweise Fette und Wachse, Perlglanzwachse, Überfettungsmittel, Stabilisatoren, kationische, zwitterionische oder amphotere Polymere, biogene Wirkstoffe, Filmbildner, Quellmittel, Hydrotrope, Konservierungsmittel, Antischuppenmittel, Selbstbräuner, Solubilisatoren, Parfümöle, Farbstoffe, etc., die nachstehend beispielhaft aufgeführt sind.

Unter Fetten und Wachsen werden im Sinne der Erfindung alle Lipide mit fett- oder wachsartiger Konsistenz verstanden, die einen Schmelzpunkt oberhalb von 20 °C aufweisen. Hierzu gehören beispielsweise die klassischen Triacylglycerine, also die Dreifachester von Fettsäuren mit Glycerin, die pflanzlicher oder tierischer Herkunft sein können. Hierbei kann es sich auch um Mischester, also um Dreifachester aus Glycerin mit verschiedenen Fettsäuren, oder aber um ein Gemisch verschiedener Glyceride handeln. Hierzu gehören auch Gemische aus Mono- Di- und Triglyceriden. Erfindungsgemäß besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett. Besonders geeignet sind oxidationsstabile pflanzliche Glyceride, die unter der Bezeichnung Cegesoft^{®} oder Novata^{®} angeboten werden.

Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Neben den Fetten kommen als Zusatzstoffe auch fettähnliche Substanzen, wie Lecithine und Phospholipide in Frage. Lecithine sind Glycero-Phospholipide, die sich aus Fettsäuren, Glycerin, Phosphorsäure und Cholin durch Veresterung bilden, und häufig auch als Phosphatidylcholine (PC) bezeichnet werden. Als Beispiel für natürliche Lecithine seien die Kephaline genannt, die auch als Phosphatidsäuren bezeichnet werden und Derivate der 1,2-Diacyl-*sn*-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate). Auch Sphingosine bzw. Sphingolipide kommen als fettartige Stoffe in Frage.

Als Perlglanzwachse geeinget sind beispielsweise Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierten Carbonsäuren mit C₆-C₂₂-Fettalkoholen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen - speziell Lauron^{®}; Distearylether; Fettsäuren wie Stearinsäure, C₁₂-C₂₂-Hydroxyfettsäuren, Behensäure, Ringöffnungsprodukte von C₁₂-C₂₂-Olefinepoxiden mit C₁₂-C₂₂-Fettalkoholen und/oder Polyolen mit 2 bis 15 Kohlenstoffatomen und 2 bis 10 Hydroxylgruppen sowie deren Mischungen.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei letztere gleichzeitig als Schaumstabilisatoren dienen.

Als sogennnte Stabilisatoren können Metallsalze von Fettsäuren, wie z.B. Magnesium-, Aluminium- und/oder Zinkstearat bzw. -ricinoleat eingesetzt werden.

Geeignete kationische Polymere, welche die Sensorik der erfindungsgemäßen Zusammensetzungen weiter optimieren und der Haut ein Gefühl der Weichheit verleihen, sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Geeignete Siliconverbindungen wurden bereits bei den Ölkörpern erwähnt. Neben Dimethylpolysiloxanen, Methylphenylpolysiloxanen und cyclischen Siliconen sind auch amino-, fettsäure-, alkohol-, polyether-, epoxy-, fluor-, glykosid- und/oder alkylmodifizierte Siliconverbindungen geeignet, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und Siliciumdioxid oder hydrierten Silicaten handelt.

Erfindungsgemäß geeignete biogene Wirkstoffe sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen. Derartige Wirkstoffe werden als Radikalfänger in Sonnenschutzformulierungen eingesetzt und dienen der Regeneration der Haut.

Sogenannte Filmbildner, die zu einer weiteren Verbesserung der Sensorik der erfindungsgemäßen Zubereitungen führen, sind beispielsweise Chitosan, mikrokristallines Chitosan, quaterniertes Chitosan, Kollagen, Hyaluronsäure bzw. deren Salze und ähnliche Verbindungen, sowie die bereits unter den Viskositätsregulatoren genannten Polyvinylpyrrolidone, Vinylpyrrolidon-Vinylacetat-Copolymerisate, Polymere der Acrylsäurereihe und quaternäre Cellulose-Derivate.

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyethylenglykolsorbitanmonooleat, Schwefelrizinolpolyethoxylat, Schwefelteer-Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion/Dipyrithion-Magnesiumsulfat in Frage.

Als Selbstbräuner eignet sich z. B. Dihydroxyaceton. Als Tyrosinaseinhibitoren, welche die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Zur Verbesserung des Fließverhaltens der erfindungsgemäßen Zusammensetzungen können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Kurzkettige Alkyglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt natürliche, pflanzliche und tierische sowie synthetische Riechstoffe oder deren Gemische. Natürliche Riechstoffe werden u.a. durch Extraktion von Blüten, Stengeln, Blättern, Früchten, Fruchtschalen, Wurzeln und Harzen von Pflanzen erhalten. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Bevorzugt werden Mischungen verschiedener Riechstoffe verwendet, die gemeinsam eine ansprechende Duftnote erzeugen.

Als Farbstoffe können die für kosmetische Zwecke geeigneten und zugelassenen Substanzen verwendet werden.

### Der Schaumspender

Die Sonnenschutzemulsionen können mit Luft oder mit Hilfe eines Treibgases aus einem Spender verschäumt werden. Zum Verschäumen der Sonnenschutzemulsion können an sich bekannte Schaumspender eingesetzt werden, wobei Spender auf Treibgasbasis oder mechanische Schaumspender mit einem Pumpenmechanismus zur Verschäumung der Sonnenschutzemulsion mit Luft zum Einsatz kommen können.
Eine Ausführungsform der Erfindung enthält die Sonnenschutzemulsion zusammen mit einem Treibgas in einem auf Treibgasbasis arbeitenden Schaumspender. Vorzugsweise liegt das Treibgas in komprimierter Form zusammen mit dem Sonnenschutzemulsion in einer Druckdose mit Sprühaufsatz vor.

Hierfür könne alle handelsüblichen Treibgasmischungen eingesetzt werden. Kohlenwasserstoffhaltige Treibgase und Treibgasgemische können bevorzugt geeignet sein, wobei ein Gehalt an C₅-Alkanen zu einem sogenannten "post-foaming" Effekt führt. Geeignet sind beispielsweise n-Pentan, 2-Methylbutan (= Isopentan) und Neopentan, wobei das 2-Methylbutan bevorzugt geeignet ist, um einen "post-foaming" Effekt zu erzeugen. Als Treibgasgemische sind auch Mischungen aus C₄-Alkanen und C₅-Alkanen einsetzbar sowie deren fluorierte Analoga.

Besonders bevorzugt ist es jedoch, dass die Sonnenschutzemulsion in einem Schaumspender mit einem Pumpenmechanismus zur Verschäumung mit Luft enthalten ist. Ein derartiger Schaumspender ermöglicht es, auf hohe Anteile flüchtiger organischer Treibgasvorläufer zu verzichten. Von Vorteil ist dies insbesondere deshalb, weil Sonnenschutzmittel häufig bei ihrer Verwendung, z. B. am Strand beim Sonnenbaden, der direkten Sonneneinstrahlung ausgesetzt und erhitzt werden.

Der einzusetzende Schaumspender soll es dem Anwender ermöglichen, problemlos, auf einfache Weise und mit hoher Sicherheit aus dem erfindungsgemäßen Sonnenschutzemulsion einen besonders intensiven und stabilen Schaum zu bilden, der nicht zusammenbricht und sich daher gut verteilen läßt.

Diese Forderung wird durch einen Schaumspender mit den nachfolgend beschriebenen Merkmalen erfüllt. Dabei kann es sich beispielsweise um einen Schaumspender handeln, der ähnlich wie der bekannte Spender nach der WO 00/78629 A1 (Airspray N.V.) aufgebaut ist, der unter der Bezeichnung F2 Finger Pump Foamer^{®} vertrieben wird.

Besonders wichtig zur Herstellung des Schaumes mit den oben genannten Eigenschaften ist das Mischungsverhältnis von Luft zu Flüssigkeit bei der Herstellung des Schaumes. Dazu wird vorgeschlagen, dass der Schaumspender ein Mischungsverhältnis von Luft zu Flüssigkeit von 5 : 1 bis 30 : 1, vorzugsweise von 8 : 1 bis 20 : 1 und insbesondere von etwa 10 : 1 aufweist.

Besonders geeignet im Hinblick auf den Gebrauch des mit der erfindungsgemäßen Sonnenschutzemulsion gefüllten Schaumspenders ist es, wenn das Dosiervolumen des Schaumspenders bei 0,1 bis 1 ml, vorzugsweise bei 0,2 bis 0,5 ml und insbesondere bei etwa 0,4 ml Flüssigkeit pro Dosierhub liegt. Die dann abgegebene Menge an Schaum lässt sich gut mit einer Hand auf eine ausreichend große Hautoberfläche verteilen, ohne dass einerseits die Schaummenge zu groß und zu unhandlich ist oder andererseits zu häufig der Schaumspender betätigt werden müsste.

Zur effektiven Erzeugung eines Schaums mit den oben genannten Eigenschaften hat es sich weiterhin als vorteilhaft herausgestellt, wenn der Schaumspender einen Auslasskanal mit einem Schaumgenerator mit mindestens einem, vorzugsweise mindestens zwei hintereinander angeordneten Flachsieben aufweist, die im Auslasskanal und im wesentlichen quer dazu angeordnet sind. In diesem Schaumgenerator wird die Mischung von Luft und Flüssigkeit durch die Siebe hindurch gedrückt und auf diese Weise der Schaum erzeugt.

Um das für die Schaumerzeugung sehr wichtige Mischungsverhältnis zwischen Luft und Flüssigkeit auch unter ungünstigen Umgebungsbedingungen zu gewährleisten, ist es von Vorteil, wenn zum einen das Eindringen von Flüssigkeit, z. B. Wasser, von außen in die Luftpumpenkammer des Schaumspender möglichst vermieden wird. Dazu wird vorgeschlagen, dass der Schaumspender mindestens einen Lufteinlass in eine Luftpumpenkammer aufweist und der Lufteinlass von einer entfernbaren Schutzkappe vor dem Eindringen von Flüssigkeit, insbesondere Wasser, geschützt ist. Falls nämlich in die Luftpumpenkammer eingedrungenes Wasser zusammen mit dem Sonnenschutzemulsion und der Luft verschäumt wird, ändert sich das vorgesehene Mischungsverhältnis zwischen Luft und Flüssigkeit in ungünstiger Weise, so dass mit einer Verschlechterung der Qualität des erzeugten Schaums zu rechnen ist.

Sollte jedoch bereits Wasser in die Luftpumpenkammer eingedrungen sein, so kann das bevorzugte Mischungsverhältnis zwischen Luft und Flüssigkeit dennoch problemlos aufrecht erhalten werden, wenn nach einer weiteren bevorzugten Ausgestaltung die Luftpumpenkammer ein von Null verschiedenes Mindestvolumen aufweist. In diesem Mindestvolumen, das auch Restvolumen genannt werden kann, kann sich eventuell eingedrungenes Wasser sammeln, so dass es beim Betätigen des Schaumspenders nicht zusammen mit dem Sonnenschutzemulsion verschäumt wird, sondern in der Luftpumpenkammer verbleibt.

### Beispiele

Die Herstellung der erfindungsgemäßen Zubereitungen erfolgte durch übliche Kalt- oder Heißprozesse. Fettphase und Wasserphase (ohne Tenside) wurden auf ca. 80 °C erwärmt-und unter Rühren zusammengegeben. Anschließend wurde unter Rühren bis auf ca. 40 °C abgekühlt und die Tenside zugefügt. Nach Zugabe der Duftstoffe bei 25 °C wurde die Emulsion homogenisiert.

Die Mengenangaben in nachfolgenden Beispielen beziehen sich, soweit nicht anders angegeben, auf Gew.-% der handelsüblichen Substanz der bevorrateten Sonnenschutzemulsion. Die Beispiele 1 bis 4 sind erfindungsgemäße Formulierungen, das Bespiel V1 dient als Vergleichsbeispiel. In Klammern ist die INCI-Nomenklatur angegeben.

### Sonnenschutzemulsion, Beispiel 1:

| | |
|---|---|
| Dehymuls^{®} PGPH (Polyglyceryl-2 Dipolyhydroxystearate) | 4,00 Gew.-% |
| Cetiol^{®} CC (Dicaprylyl Carbonate) | 5,00 Gew.-% |
| Myritol^{®} 312 (Caprylic/Capric Triglyceride) | 3,00 Gew.-% |
| Neo Heliopan^{®} MBC (4-Methylbenzylidene Camphor) | 4,00 Gew.-% |
| Neo Heliopan^{®} OS (Ethylhexyl Salicylate) | 2,00 Gew.-% |
| Parsol^{®} 1789 (Butyl Methoxydibenzoylmethane) | 2,00 Gew.-% |
| Copherol^{®} 1250 (Tocopheryl Acetate) | 0,60 Gew.-% |
| Stay-C^{®} 50 (Sodium Ascorbyl Phosphate) | 0,10 Gew.-% |
| Neo Heliopan^{®} Hydro (Phenylbenzimidazole Sulfonic Acid) | 1,00 Gew.-% |
| Natriumydroxid - | 0,20 Gew.-% |
| Rewopol^{®} SB CS 50K (Disodium PEG-5 Laurylcitrate Sulfosuccinate, Sodium Laureth Sulfate) | 2,00 Gew.% |
| Tego^{®} Betain 810 (Capryl/Capramidopropyl Betaine) | 3,00 Gew.-% |
| Konservierungsmittel | q.s. |
| Parfüm | q.s. |
| Wasser | ad 100 Gew.-% |

### Sonnenschutzemulsion, Beispiel 2:

| | |
|---|---|
| Dehymuls^{®} PGPH (Polyglyceryl-2 Dipolyhydroxystearate) | 4,00 Gew.-% |
| Cetiol^{®} CC (Dicaprylyl Carbonate) | 5,00 Gew.-% |
| Myritol^{®} 331 (Cocoglycerides) | 3,00 Gew.-% |
| Neo Heliopan^{®} MBC (4-Methylbenzylidene Camphor) | 4,00 Gew.-% |
| Neo Heliopan^{®} OS (Ethylhexyl Salicylate) | 2,00 Gew.-% |
| Uvasorb^{®} HEB (Diethylhexyl Butamido Triazone) | 3,00 Gew.-% |
| Parsol^{®} 1789 (Butyl Methoxydibenzoylmethane) | 2,00 Gew.-% |
| Copherol^{®} 1250 (Tocopheryl Acetate) | 0,60 Gew.-% |
| Stay-C^{®} 50 (Sodium Ascorbyl Phosphate) | 0,10 Gew.-% |
| Neo Heliopan^{®} Hydro (Phenylbenzimidazole Sulfonic Acid) | 1,00 Gew.-% |
| Natriumhydroxid | 0,20 Gew.-% |
| Rewopol^{®} SB CS 50K (Disodium PEG-5 Laurylcitrate Sulfosuccinate, Sodium Laureth Sulfate) | 2,00 Gew.% |
| Tego^{®} Betain 810 (Capryl/Capramidopropyl Betaine) | 3,00 Gew.-% |
| Konservierungsmittel | q.s. |
| Parfüm | q.s. |
| Wasser | ad 100 Gew.-% |

### Sonnenschutzemulsion, Beispiel 3:

| | |
|---|---|
| Dehymuls^{®} PGPH (Polyglyceryl-2 Dipolyhydroxystearate ) | 4,00 Gew.-% |
| Cetiol^{®} CC (Dicaprylyl Carbonate) | 5,00 Gew.-% |
| Myritol^{®} 331 (Cocoglycerides) | 3,00 Gew.-% |
| Neo Heliopan^{®} MBC (4-Methylbenzylidene Camphor) | 4,00 Gew.-% |
| Parsol^{®} SLX (Benzylidene Malonate Polysiloxane) | 2,00 Gew.-% |
| Uvasorb^{®} HEB (Diethylhexyl Butamido Triazone) | 3,00 Gew.-% |
| Parsol^{®} 1789 (Butyl Methoxydibenzoylmethane) | 2,00 Gew.-% |
| Copherol^{®} 1250 (Tocopheryl Acetate) | 0,60 Gew.-% |
| Stay-C^{®} 50 (Sodium Ascorbyl Phosphate) | 0,10 Gew.-% |
| Neo Heliopan^{®} Hydro (Phenylbenzimidazole Sulfonic Acid) | 1,00 Gew.-% |
| Natriumhydroxid | 0,20 Gew.-% |
| Rewopol^{®} SB CS 50K (Disodium PEG-5 Laurylcitrate Sulfosuccinate, Sodium Laureth Sulfate) | 2,00 Gew.% |
| Tego^{®} Betain 810 (Capryl/Capramidopropyl Betaine) | 3,00 Gew.-% |
| Konservierungsmittel | q.s. |
| Parfüm | q.s. |
| Wasser | ad 100 Gew.-% |

### Sonnenschutzemulsion, Beispiel 4:

| | |
|---|---|
| Dehymuls^{®} PGPH (Polyglyceryl-2 Dipolyhydroxystearate) | 4,00 Gew.-% |
| Cetiol^{®} OE (Dicaprylyl Ether) | 2,00 Gew.-% |
| Finsolv^{®} TN (C12/15 Alkyl Benzoate) | 3,00 Gew.-% |
| Cegesoft^{®} C 24 (Ethylhexyl Palmitate) | 3,00 Gew.-% |
| Neo Heliopan^{®} MBC (4-Methylbenzylidene Camphor) | 4,00 Gew.-% |
| Neo Heliopan^{®} OS (Ethylhexyl Salicylate) | 2,00 Gew.-% |
| Neo Heliopan^{®} AP (Disodium Phenyl Dibenzimidazole Tetrasulfonate) | 3,00 Gew.-% |
| Copherol^{®} 1250 (Tocopheryl Acetate) | 0,60 Gew.-% |
| Stay-C^{®} 50 (Sodium Ascorbyl Phosphate) | 0,10 Gew.-% |
| Neo Heliopan^{®} Hydro (Phenylbenzimidazole Sulfonic Acid) | 1,00 Gew.-% |
| Natriumydroxid | 0,20 Gew.-% |
| Rewopol^{®} SB CS 50K (Disodium PEG-5 Laurylcitrate Sulfosuccinate, Sodium Laureth Sulfate) | 2,00 Gew.% |
| Tego^{®} Betain 810 (Capryl/Capramidopropyl Betaine) | 3,00 Gew.-% |
| Glycerin | 3,00 Gew.-% |
| Konservierungsmittel | q.s. |
| Parfüm | q.s. |
| Wasser | ad 100 Gew.-% |

### Sonnenschutzemulsion, Vergleichsbeispiel 1 (nicht verschäumbar):

| | |
|---|---|
| Rylo^{®} PG 19 (Polyglyceryl-3 Polyricinoleate) | 4,00 Gew.-% |
| Cetiol^{®} CC (Dicaprylyl Carbonate) | 5,00 Gew.-% |
| Myritol^{®} 331 (Cocoglycerides) | 3,00 Gew.-% |
| Neo Heliopan^{®} MBC (4-Methylbenzylidene Camphor) | 4,00 Gew.-% |
| Neo Heliopan^{®} OS (Ethylhexyl Salicylate) | 2,00 Gew.-% |
| Uvasorb^{®} HEB (Diethylhexyl Butamido Triazone) | 3,00 Gew.-% |
| Parsol^{®} 1789 (Butyl Methoxydibenzoylmethane) | 2,00 Gew.-% |
| Copherol^{®} 1250 (Tocopheryl Acetate) | 0,60 Gew.-% |
| Stay-C^{®} 50 (Sodium Ascorbyl Phosphate) | 0,10 Gew.-% |
| Neo Heliopan^{®} Hydro (Phenylbenzimidazole Sulfonic Acid) | 1,00 Gew.-% |
| Natriumhydroxid | 0,20 Gew.-% |
| Rewopol^{®} SB CS 50K (Disodium PEG-5 Laurylcitrate Sulfosuccinate, Sodium Laureth Sulfate) | 2,00 Gew.% |
| Tego^{®} Betain 810 (Capryl/Capramidopropyl Betaine) | 3,00 Gew.-% |
| Konservierungsmittel | q.s. |
| Parfüm | q.s. |
| Wasser | ad 100 Gew.-% |

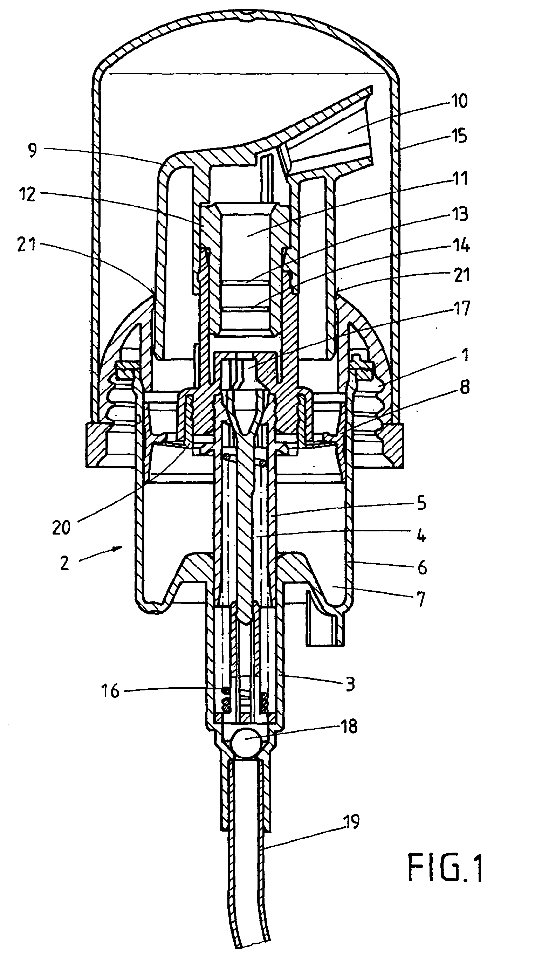

### Beispiel Schaumspender (Figur 1):

Die einzige Figur zeigt einen Längsschnitt durch einen Schaumspender mit einem Pumpenmechanismus der Fa. Airspray International B.V., welcher besonders gut zum Verschäumen und Dosieren der erfindungsgemäßen Sonnenschutzemulsion geeignet ist. Die Figur zeigt den Schaumspender ohne den von unten anzuschraubenden Flüssigkeitsbehälter und in einer Position vor dem Betätigen des Pumpenmechanismus. Im zusammengesetzten Zustand wird der Flüssigkeitsbehälter, dessen Mündungsöffnung ein Außengewinde aufweist, in das Innengewinde 1 der in Figur 1 dargestellten Schaumpumpe 2 eingeschraubt. Der Schaumspender besteht also aus der Schaumpumpe 2 und dem nicht dargestellten Flüssigkeitsbehälter für die Sonnenschutzemulsion.

Die Schaumpumpe 2 enthält eine Flüssigkeitspumpe 3 mit einer Pumpkammer 4 und einem Pumpkolben 5 für die Flüssigkeit. Weiterhin ist eine Luftpumpe 6 mit einer Luftpumpenkammer 7 und einem Luftpumpenkolben 8 vorgesehen. Beide Kolben, nämlich der Flüssigkeitspumpenkolben 5 und der Luftpumpenkolben 8 sind mechanisch mit einem Betätigungskopf 9 verbunden.

Der Betätigungskopf 9 weist eine schräg nach oben gerichtete Auslassöffnung 10 für den Schaum und einen in-Richtung-der Längsachse des Schaumspenders, in Figur 1 also lotrecht, ausgerichteten Auslasskanal 11 auf, in welchem ein Schaumgenerator 12 eingesetzt ist. Der Schaumgenerator 12 besteht aus einer Hülse mit zwei quer zur Längsachse eingesetzten Flachsieben 13,14. Der Betätigungskopf 9 ist schließlich von einer entfernbaren Schutzkappe 15 umgeben.

Die übrigen wesentlichen Teile dieses Schaumspenders werden nachfolgend bei der Beschreibung der Funktionsweise erläutert. Zum Betätigen nimmt der Verbraucher die Schutzkappe 15 ab und drückt von oben auf den Betätigungskopf 9, so dass dieser sich nach unten gegen die Kraft einer Druckfeder 16 bewegt. Dabei gleiten der Pumpkolben 5 für die Flüssigkeit in seiner Pumpkammer 4 und der Luftpumpenkolben 8 in der Luftpumpenkammer 7 nach unten, so dass die jeweiligen Volumina der entsprechenden Pumpkammern 4, 7 verringert werden und sowohl Luft als auch Flüssigkeit in eine Mischkammer 17 gelangen, in der Luft und Flüssigkeit gemischt werden. Die Mischung strömt dann durch die Flachsiebe 13 und 14, wobei ein Schaum erzeugt wird, der dann über den Auslasskanal 11 und die Auslassöffnung 10 nach außen abgegeben wird.

Nach der Abgabe des Schaums lässt der Anwender den Betätigungskopf 9 los, der dann durch die Kraft der Druckfeder 16 in seine in Figur 1 dargestellte Anfangsposition zurückkehrt. Während dieser Rückbewegung öffnet sich ein Rückschlagventil 18, dessen Ventilglied als Kugel ausgebildet ist, und die Pumpkammer 4 für die Flüssigkeit füllt sich mit der Sonnenschutzemulsion, da diese aus dem in Figur 1 nicht dargestellten Flüssigkeitsbehälter über ein Röhrchen 19 angesaugt wird. Gleichzeitig öffnet sich eine ringförmige flexible Ventildichtung 20 der Luftpumpenkammer 7, um Luft von außen durch einen Lufteinlass 21 in die Luftpumpenkammer 7 anzusaugen.

Hat der Betätigungskopf 9 seine obere Position entsprechend der Darstellung in Figur 1 erreicht, kann der Schaumspender erneut durch Herunterdrücken des Betätigungskopfes 9 schaumförmige Sonnenschutzemulsion erzeugen und abgeben. Bei diesem Herunterdrücken schließen das Rückschlagventil 18 für den Einlass der Flüssigkeits-Pumpkammer 4 und die Ventildichtung 20 für die Luftpumpenkammer 7, so dass die Flüssigkeit und die Luft nur durch vorgegebene Passagen in die Mischkammer 17 gelangen können.

Das für die Schaumerzeugung sehr wichtige Mischungsverhältnis zwischen Luft und Flüssigkeit liegt bei der Pumpe in diesem bevorzugten Ausführungsbeispiel bei etwa 10 : 1. Bei jeder Betätigung werden etwa 0,4 ml Flüssigkeit in Form von Schaum abgegeben. Eine derartige Pumpe ist unter der Bezeichnung M3 von der Fa. Airspray International B.V., Alkmaar, Niederlande erhältlich.

Um das genannte Mischungsverhältnis zwischen Luft und Sonnenschutzemulsion unter möglichst allen Anwendungsbedingungen zu gewährleisten, ist es weiterhin von Vorteil, wenn der Lufteinlass 21 in die Luftpumpenkammer 7 vor einem Eindringen von Flüssigkeit, z. B. Wasser, von außen möglichst gut geschützt ist. Bei einem Eindringen von Wasser in die Luftpumpenkammer 7 könnte dieses Wasser nämlich zusammen mit der Luft in die Mischkammer 17 gedrückt werden, so dass sich das Mischungsverhältnis von Luft zu Flüssigkeit in ungünstiger Weise verändert und die Qualität des erzeugten Schaums verschlechtert.

Ein gewisser Schutz gegen das Eindringen von Wasser bei nicht benutztem Schaumspender bietet die Schutzkappe 15. Ein weiterer Schutz gegen eine Verschlechterung des Mischungsverhältnisses durch Eindringen von Wasser in die Luftpumpenkammer 7 ist bei der Pumpe nach dem Ausführungsbeispiel auch dadurch gegeben, dass die Stirnseite des Luftpumpenkolbens 8 und die Form des Bodens der Luftpumpenkammer 7 sich nicht entsprechen. Daher verbleibt bei einem vollständigen Herunterdrücken des Betätigungskopfes 9 immer ein gewisses Restvolumen (Mindestvolumen) in der Luftpumpenkam= mer 7, welches im Ausführungsbeispiel die Form eines Ringraumes hat, in welchem sich eventuell eingedrungenes Wasser sammeln kann, ohne dass es zusammen mit der Luft in die Mischkammer 17 transportiert wird.

### Bezugszeichenliste

- 1: Innengewinde
- 2: Schaumpumpe
- 3: Flüssigkeitspumpe
- 4: Pumpkammer für Flüssigkeit
- 5: Pumpkolben für Flüssigkeit
- 6: Luftpumpe
- 7: Luftpumpenkammer
- 8: Luftpumpenkolben
- 9: Betätigungskopf
- 10: Auslassöffnung
- 11: Auslasskanal
- 12: Schaumgenerator
- 13: Flachsieb
- 14: Flachsieb
- 15: Schutzkappe
- 16: Druckfeder
- 17: Mischkammer
- 18: Rückschlagventil
- 19: Röhrchen
- 20: Ventildichtung
- 21: Lufteinlass

### Anhang

1) Cegesoft® C 24
   INCI: Ethylhexyl Palpitate
   Hersteller: Cognis Deutschland GmbH & Co. KG
2) Cetiol^{®} CC
   INCI: Dicaprylyl Carbonate
   Hersteller: Cognis Deutschland GmbH & Co. KG
3) Copherol® 1250
   INCI: Tocopheryl Acetate
   Hersteller: Cognis Corporation
4) Dehymuls^{®} PGPH
   INCI: Polyglyceryl-2 Dipolyhydroxystearate
   Hersteller: Cognis Deutschland GmbH & Co. KG
5) Finsolv® TN
   INCI: C12/15 Alkyl Benzoate
   Hersteller: Nordmann Rassmann
6) Myritol® 312
   INCI: Caprylic/Capric Triglyceride
   Hersteller: Cognis Deutschland GmbH & Co. KG
7) Myritol® 331
   INCI: Cocoglycerides
   Hersteller: Cognis Deutschland GmbH & Co. KG
8) Neo Heliopan® AP
   INCI: Disodium Phenyl Dibenzimidazole Tetrasulfonate
   Hersteller: Haarmann & Reimer
9) Neo Heliopan^{®} MBC
   INCI: 4-Methylbenzylidene Camphor
   Hersteller: Haarmann & Reimer
10) Neo Heliopan® OS
   INCI: Ethylhexyl Salicylate
   Hersteller: Haarmann & Reimer
11) Neo Heliopan® Hydro
   INCI: Phenylbenzimidazole Sulfonic Acid
   Hersteller: Haarmann & Reimer
12) Parsol® 1789
   INCI: Butyl Methoxydibenzoylmethane
   Hersteller: Hoffmann-La Roche (Givaudan)
13) Parsol® SLX
   Benzylidene Malonate Polysiloxane
   Hersteller: Hoffmann-La Roche
14) Rylo® PG 19
   INCI: Polyglycery(-3 Polyricinoleate
   Hersteller: Danisco Cultor
15) Rewopol® SB CS 50K
   INCI: Disodium PEG-5 Laurylcitrate Sulfosuccinate, Sodium Laureth Sulfate
   Hersteller: Goldschmidt-Rewo
16) Stay-C^{®} 50
   INCI: Sodium Ascorbyl Phosphate
   Hersteller: Hoffmann-La Roche
17) Tego® Betain 810
   INCI: Capryl/Capramidopropyl Betaine
   Hersteller: Goldschmidt-Rewo
18) Uvasorb® HEB
   INCI: Diethylhexyl Butamido Triazone
   Hersteller: Sigma

## Patentansprüche

1. System aus einem manuell betätigbarem Schaumspender und einer Sonnenschutzemulsion, **gekennzeichnet durch** einen Gehalt an:
(a) 2-10 Gew.-% wenigstens eines Poly(12-hydroxystearinsäure)polyglycerinesters
(b) Ölkörper, enthaltend Dialkylcarbonate und/oder Dialkylether
(c) weitere Tenside
(d) UV-Lichtschutzfilter und
(e) Wasser

2. System aus einem manuell betätigbarem Schaumspender und einer Sonnenschutzemulsion, **dadurch gekennzeichnet, dass** die Sonnenschutzemulsion
(a) 2 - 10 Gew.-% Polyolpoly-12-Hydroxystearate
(b) 1 - 20 Gew.-% Ölkörper, enthaltend Dialkylcarbonate und/oder Dialkylether
(c) 0,5 - 10 Gew.-% weitere Tenside
(d) 0,5 - 20 Gew.-% UV-Lichtschutzfilter und
(e) 30 - 80 Gew.-% Wasser
enthält.

3. System gemäß Anspruche 2, **dadurch gekennzeichnet, dass** als Polyolpoly-12-Hydroxystearate (a) wenigstens ein Poly(12-hydroxystearinsäure)polyglycerinester enthalten ist.

4. System gemäß wenigstens einem der Ansprüche 1 bis 3, **dadurch gekennzeichnet, dass** als Tenside (c) wenigstens ein Tensid ausgewählt aus der Gruppe der Aniontenside und/oder der zwitterionischen Tenside enthalten ist.

5. System gemäß wenigstens einem der Ansprüche 1 bis 4, **dadurch gekennzeichnet, dass** die Sonnenschutzemulsion
(a) 2 - 10 Gew.-% Poly(12-hydroxystearinsäure)polyglycerinester
(b) 1 - 20 Gew.-% Ölkörper enthaltend Dialkylcarbonate
(c) 0,5 - 10 Gew.-% eines Gemisches aus Cocamidopropylbetain und Sulfosuccinaten
(d) 0,5 - 20 Gew.-% UV-Lichtschutzfilter und
(e) 30 - 80 Gew.-% Wasser
enthält.

6. System gemäß wenigstens einem der Ansprüche 1 bis 5, **dadurch gekennzeichnet, dass** die Sonnenschutzemulsion eine W/O-Emulsion ist.

7. System gemäß wenigstens einem der Ansprüche 1 bis 6, **dadurch gekennzeichnet, dass** die Sonnenschutzemulsion zusammen mit einem Treibgas in einem auf Treibgasbasis arbeitenden Schaumspender enthalten ist.

8. System gemäß wenigstens einem der Ansprüche 1 bis 7, **dadurch gekennzeichnet, dass** die Sonnenschutzemulsion in einem Schaumspender mit einem Pumpenmechanismus zur Verschäumung mit Luft enthalten ist.

9. System gemäß Anspruch 8, **dadurch gekennzeichnet, dass** der Schaumspender ein Mischungsverhältnis von Luft zu Flüssigkeit von 5 : 1 bis 30 : 1, vorzugsweise von 8 : 1 bis 20 : 1 und insbesondere von etwa 10 : 1 aufweist.

10. System gemäß einem der Ansprüche 8 bis 9, **dadurch gekennzeichnet, dass** das Dosiervolumen des Schaumspenders bei 0,1 bis 1 ml, vorzugsweise bei 0,2 bis 0,5 ml und insbesondere bei etwa 0,4 ml Flüssigkeit pro Dosierhub liegt.

11. System gemäß wenigstens einem der Ansprüche 8 bis 10, **dadurch gekennzeichnet, dass** der Schaumspender einen Auslasskanal (11) mit einem Schaumgenerator (12) mit mindestens einem, vorzugsweise mindestens zwei hintereinander angeordneten Flachsieben (13, 14) aufweist, die im Auslasskanal (11) und im Wesentlichen quer dazu angeordnet sind.

12. System gemäß wenigstens einem der Ansprüche 8 bis 11, **dadurch gekennzeichnet, dass** der Schaumspender mindestens einen Lufteinlass (21) in eine Luftpumpenkammer (7) aufweist und der Lufteinlass (21) von einer entfernbaren Schutzkappe (15) vor dem Eindringen von Flüssigkeit, insbesondere Wasser, geschützt ist.

13. System gemäß wenigstens einem der Ansprüche 8 bis 12, **dadurch gekennzeichnet, dass** der Schaumspender eine Luftpumpenkammer (7) und die Luftpumpenkammer (7) ein von Null verschiedenes Mindestvolumen aufweist.

14. Sonnenschutzemulsion **gekennzeichnet durch** einen Gehalt an:
(a) 2-10 Gew.-% wenigstens eines Poly(12-hydroxystearinsäure)polyglycerinesters
(b) Ölkörpern, enthaltend Dialkylcarbonate und/oder Dialkylether
(c) weiteren Tensiden
(d) UV-Lichtschutzfiltern und
(e) Wasser,
wobei als Tenside (c) wenigstens ein Tensid ausgewählt aus der Gruppe der Aniontenside und/oder der zwitterionischen Tenside enthalten ist.

15. Sonnenschutzemulsion **dadurch gekennzeichnet, daß** sie
(a) 2-10 Gew.-% Polyolpoly-12-Hydroxystearate
(b) 1 - 20 Gew.-% Ölkörper, enthaltend Dialkylcarbonate und/oder Dialkylether
(c) 0,5 -10 Gew.-% weitere Tenside und
(d) 0,5 - 20 Gew.-% UV-Lichtschutzfilter
(e) 30 - 80 Gew.-% Wasser
enthält.

16. Sonnenschutzemulsion gemäß Anspruch 15, **dadurch gekennzeichnet, dass** als Polyolpoly-12-Hydroxystearate (a) wenigstens ein Poly(12-hydroxystearinsäure)polyglycerinester enthalten ist.

17. Sonnenschutzemulsion gemäß wenigstens einem der Ansprüche 15 bis 16, **dadurch gekennzeichnet, dass** als Tenside (c) wenigstens ein Tensid ausgewählt aus der Gruppe der Aniontenside und/oder der zwitterionischen Tenside enthalten ist.

18. Sonnenschutzemulsion gemäß wenigstens einem der Ansprüche 14 bis 17, **dadurch gekennzeichnet, dass** sie
(a) 2 - 10 Gew.-% Poly(12-hydroxystearinsäure)polyglycerinester
(b) 1- 20 Gew.-% Ölkörper
(c) 0,5-10 Gew.-% eines Gemisches aus Cocamidopropylbetain und Sulfosuccinaten und
(d) 0,5 - 20 Gew.-% UV-Lichtschutzfilter
(e) 30-80 Gew.-% Wasser
enthält.

19. Sonnenschutzemulsion gemäß wenigstens einem der Ansprüche 14 bis 18, **dadurch gekennzeichnet, dass** sie eine W/O-Emulsion ist.

20. Verwendung von Polyolpoly-12-Hydroxystearaten, insbesondere von Poly(12-hydroxystearinsäure)polyglycerinestem, zur Verbesserung der Verschäumbarkeit von Emulsionen in Schaumspendern.

## Claims

1. A system of a manually operated foam dispenser and a sun protection emulsion **characterized by** a content of:
(a) 2-10 weight-% of at least one poly(12-hydroxystearic acid)polyglycerol ester
(b) oil components, containing dialkyl carbonates and/or dialkyl ethers
(c) other surfactants
(d) UV protection factors and
(e) water.

2. A system of a manually operated foam dispenser and a sun protection emulsion, **characterized in that** the sun protection emulsion contains
(a) 2 to 10% by weight polyol poly-12-hydroxystearates
(b) 1 to 20% by weight oil components, containing dialkyl carbonates and/or dialkyl ethers
(c) 0.5 to 10% by weight other surfactants
(d) 0.5 to 20% by weight UV protection factors and
(e) 30 to 80% by weight water.

3. A system as claimed in claim 2, **characterized in that** at least one poly(12-hydroxystearic acid)polyglycerol ester is present as the polyol poly-12-hydroxystearate (a).

4. A system as claimed in at least one of claims 1 to 3, **characterized in that** at least one surfactant selected from the group of anionic surfactants and/or zwitterionic surfactants is present as the surfactant (c).

5. A system as claimed in at least one of claims 1 to 4, **characterized in that** the sun protection emulsion contains
(a) 2 to 10% by weight poly(12-hydroxystearic acid)polyglycerol ester
(b) 1 to 20% by weight oil components containing dialkyl carbonates
(c) 0.5 to 10% by weight of a mixture of Cocamidopropylbetaine and sulfosuccinates
(d) 0.5 to 20% by weight sun protection factors and
(e) 30 to 80% by weight water.

6. A system as claimed in at least one of claims 1 to 5, **characterized in that** the sun protection emulsion is a w/o emulsion.

7. A system as claimed in at least one of claims 1 to 6, **characterized in that** the sun protection emulsion is accommodated together with a blowing gas in a foam dispenser operating on the basis of a blowing gas.

8. A system as claimed in at least one of claims 1 to 7, **characterized in that** the sun protection emulsion is accommodated in a foam dispenser with a pump mechanism for foaming with air.

9. A system as claimed in claim 8, **characterized in that** the foam dispenser has an air to liquid mixing ratio of 5:1 to 30:1, preferably 8:1 to 20:1 and, more particularly, ca. 10:1.

10. A system as claimed in claim 8 or 9, **characterized in that** the foam dispenser has a shot volume of 0.1 to 1 ml, preferably 0.2 to 0.5 ml and, more particularly, ca. 0.4 ml liquid per shot.

11. A system as claimed in at least one of claims 8 to 10, **characterized in that** the foam dispenser has an outlet channel (11) with a foam generator (12) comprising at least one and preferably two flat sieves (13,14) arranged in tandem which are disposed in and substantially transversely of the outlet passage (11).

12. A system as claimed in at least one of claims 8 to 11, **characterized in that** the foam dispenser has at least one air inlet (21) into an air pump chamber (7) and **in that** the air inlet (21) is protected against the penetration of liquid, especially water, by a removable protective cap (15).

13. A system as claimed in at least one of claims 8 to 12, **characterized in that** the foam dispenser has an air pump chamber (7) and the air pump chamber (7) has a minimum volume different from zero.

14. A sun protection emulsion, **characterized by** a content of
(a) 2-10 weight-% of at least one poly(12-hydroxystearic acid)polyglycerol ester
(b) oil components, containing dialkyl carbonates and/or dialkyl ethers
(c) other surfactants
(d) UV protection factors and
(e) water
wherein at least one surfactant selected from the group of anionic surfactants and/or zwitterionic surfactants is present as the surfactant (c).

15. A sun protection emulsion, **characterized in that** contains
(a) 2 to 10% by weight polyol poly-12-hydroxystearates
(b) 1 to 20% by weight oil components, containing dialkyl carbonates and/or dialkyl ethers
(c) 0.5 to 10% by weight other surfactants
(d) 0.5 to 20% by weight UV protection factors and
(e) 30 to 80% by weight water.

16. A sun protection emulsion as claimed in claim 15, **characterized in that** at least one poly(12-hydroxystearic acid)polyglycerol ester is present as the polyol poly-12-hydroxystearate (a).

17. A sun protection emulsion as claimed in at least one of claims 15 to 16, **characterized in that** at least one surfactant selected from the group of anionic surfactants and/or zwitterionic surfactants is present as the surfactant (c).

18. A sun protection emulsion as claimed in at least one of claims 14 to 17, **characterized in that** it contains
(a) 2 to 10% by weight poly(12-hydroxystearic acid)polyglycerol ester
(b) 1 to 20% by weight oil components
(c) 0.5 to 10% by weight of a mixture of Cocamidopropylbetaine and sulfosuccinates
(d) 0.5 to 20% by weight sun protection factors and
(e) 30 to 80% by weight water.

19. A sun protection emulsion as claimed in at least one of claims 14 to 18, **characterized in that** it is a w/o emulsion.

20. The use of polyol poly-12-hydroxystearates, more particularly poly(12-hydroxystearic acid)polyglycerol esters, for improving the foamability of emulsions in foam dispensers.

## Revendications

1. Système constitué d'un distributeur de mousse à actionnement manuel et d'une émulsion de protection solaire,
**caractérisé en ce qu'**
il contient de
(a) 2 à 10 % en poids d'au moins un ester polyglycérique d'acide poly-(12-hydroxystéarique),
(b) un corps huileux contenant des carbonates de dialkyle et/ou des éthers dialkyliques,
(c) d'autres tensioactifs,
(d) un filtre photoprotecteur anti-UV, et
(e) de l'eau.

2. Système constitué d'un distributeur de mousse à actionnement manuel et d'une émulsion protection solaire,
**caractérisé en ce que**
l'émulsion protection solaire contient :
(a) de 2 à 10 % en poids de poly-12-hydroxystéarates de polyol,
(b) de 1 à 20 % en poids de corps huileux contenant des carbonates de dialkyle et/ou des éthers dialkyliques,
(c) de 0,5 à 10 % en poids d'autres tensioactifs,
(d) de 0,5 à 20 % en poids de filtre photoprotecteur anti-UV, et
(e) de 30 à 80 % en poids d'eau.

3. Système selon la revendication 2
**caractérisé en ce que**
il contient comme poly-12-hydroxystéarates de polyol (a) au moins un ester polyglycérique d'acide poly(12-hydroxystéarique).

4. Système selon au moins l'une des revendications 1 à 3,
**caractérisé en ce qu'**
il contient comme tensioactifs (c) au moins un tensioactif choisi dans le groupe des tensioactifs anioniques et/ou des tensioactifs zwitterioniques.

5. Système selon au moins l'une des revendications 1 à 4,
**caractérisé en ce que**
l'émulsion de protection solaire contient :
(a) de 2 à 10 % en poids d'ester polyglycérique d'acide poly(12-hydroxystéarique),
(b) de 1 à 20 % en poids de corps huileux contenant des carbonates de dialkyle,
(c) de 0,5 à 10 % en poids d'un mélange constitué de cocamidopropylbétaïne et de sulfosuccinates,
(d) de 0,5 à 20 % en poids de filtre photoprotecteur anti-UV, et
(e) de 30 à 80 % en poids d'eau.

6. Système selon au moins l'une des revendications 1 à 5,
**caractérisé en ce que**
l'émulsion de protection solaire est une émulsion E/H.

7. Système selon au moins l'une des revendications 1 à 6,
**caractérisé en ce qu'**
il contient l'émulsion de protection solaire ainsi qu'un gaz propulseur, dans un distributeur de mousse fonctionnant sous l'action du gaz propulseur.

8. Système selon au moins l'une des revendications 1 à 7,
**caractérisé en ce que**
l'émulsion de protection solaire est contenue dans un distributeur de mousse comportant un mécanisme de pompe lui permettant de mousser avec de l'air.

9. Système selon la revendication 8,
**caractérisé en ce que**
le distributeur de mousse présente un rapport de mélange air/liquide de 5:1 à 30:1, de préférence de 8:1 à 20:1 et, en particulier d'environ 10:1.

10. Système selon l'une des revendications 8 et 9,
**caractérisé en ce que**
le volume dosé du distributeur de mousse est de 0,1 à 1 ml, de préférence de 0,2 à 0,5 ml et en particulier d'environ 0,4 ml de liquide par course de dosage.

11. Système selon au moins l'une quelconque des revendications 8 à 10, **caractérisé en ce que**
le distributeur de mousse comporte un canal de sortie (11) muni d'un générateur de mousse (12) avec au moins un, de préférence au moins deux tamis plats (13, 14), disposés l'un derrière l'autre et placés dans le canal de sortie (11) essentiellement transversalement à celui-ci.

12. Système selon au moins l'une des revendications 8 à 11,
**caractérisé en ce que**
le distributeur de mousse comporte au moins une prise d'air (21) dans une chambre de pompage d'air (7), et la prise d'air (21) est protégée par un capuchon de protection (15) amovible contre la pénétration de liquide, en particulier d'eau.

13. Système selon au moins l'une des revendications 8 à 12,
**caractérisé en ce que**
le distributeur de mousse comporte une chambre de pompage d'air (7), et la chambre de pompage d'air (7) comporte un volume minimum différent de zéro.

14. Emulsion de protection solaire
**caractérisée en ce qu'**
elle contient,
(a) de 2 à 10 % en poids d'au moins un ester polyglycérique d'acide poly(12-hydroxystéarique),
(b) des corps huileux contenant des carbonates de dialkyle et/ou des éthers dialkyliques,
(c) d'autres tensioactifs,
(d) des filtres photoprotecteurs anti-UV, et
(e) de l'eau,
les tensioactifs (c), contenant au moins un tensioactif choisi dans le groupe des tensioactifs anioniques et/ou des tensioactifs zwitterioniques.

15. Emulsion de protection solaire,
**caractérisée en ce qu'**
elle contient :
(a) de 2 à 10 % en poids de poly-12-hydroxystéarates de polyol,
(b) de 1 à 20 % en poids de corps huileux contenant des carbonates de dialkyle et/ ou des éthers dialkyliques,
(c) de 0,5 à 10 % en poids d'autres tensioactifs,
(d) de 0,5 à 20 % en poids de filtre photoprotecteur anti-UV, et
(e) de 30 à 80 % en poids d'eau.

16. Emulsion de protection solaire selon la revendication 15,
**caractérisée en ce que**
comme poly-12-hydroxystéarates de polyol (a) elle contient au moins un ester polyglycérique d'acide poly-12-hydroxystéarique.

17. Emulsion de protection solaire selon au moins l'une des revendications 15 à 16,
**caractérisée en ce que**
comme tensioactifs (c) elle contient au moins un tensioactif choisi dans le groupe des tensioactifs anioniques et/ou des tensioactifs zwitterioniques.

18. Emulsion de protection solaire selon au moins l'une des revendications 14 à 17,
**caractérisée en ce qu'**
elle contient
(a) de 2 à 10 % en poids d'ester polyglycérique d'acide poly(12-hydroxystéarique),
(b) de 1 à 20 % en poids de corps huileux,
(c) de 0,5 à 10 % en poids d'un mélange de cocamidopropylbétaïne, et de sulfosuccinates, et
(d) de 0,5 à 20 % en poids de filtre photoprotecteur anti-UV, et
(e) de 30 à 80 % en poids d'eau.

19. Emulsion de protection solaire selon au moins l'une des revendications 14 à 18,
**caractérisée en ce qu'**
elle est constituée par une émulsion E/H.

20. Utilisation de poly-12-hydroxystéarates de polyol, en particulier d'esters polyglycériques d'acide poly(12-hydroxystéarique), pour améliorer le pouvoir moussant d'émulsions dans des distributeurs de mousse.
